# EUROPEAN PATENT APPLICATION

(11) **EP 4 005 514 A1**
(43) Date of publication of application: **01.06.2022**
(21) Application number: 21210524.1
(22) Date of filing: 25.11.2021
(51) Int. Cl.: A61B 17/42

(54) **MANIPULATOR**

(30) Priority: 30.11.2020 PL 43617220
(71) Applicant: Szpital Specjalistyczny Pro-Familia Tomasz Lozinski Spolka Komandytowa, 35-302 Rzeszow (PL)
(72) Inventor: JOPEK, Wojciech, 54-072 Wroclaw (PL)
(74) Representative: AOMB Polska Sp. z.o.o.

(57) **Abstract**

The invention relates to a vaginal and uterine manipulator applicable to the displacement of the vagina and uterus during magnetic resonance imaging and/or focused ultrasound thermal ablation treatments.

A manipulator includes an insert (1), a base (2) and a displacement mechanism (3). The displacement mechanism (3) enables the vertical position of the insert (1) and the angle between the axis of the insert and the patient's sagittal plane to be adjusted. The displacement mechanism (3) comprises an at least one vertical member (4), a main member (5), an at least two positioning pins (6), a vertical pusher, an angular pusher, a first arm (7), a second arm (8), a third arm (9), a fourth arm (10). The above-described mechanism allows to maintain a constant point of rotation (15) of the insert (1).

## Description

### Technical field

The invention relates to a vaginal and uterine manipulator applicable to the displacement of the vagina and uterus during magnetic resonance imaging and/or focused ultrasound thermal ablation treatments.

### Background

Various types of manipulators for displacing the vagina or uterus are known in the art.

US20090222025A1 discloses devices, systems and method for retracting, lifting, compressing, supporting or repositioning tissues, organs, anatomical structures, grafts or other structures within the body of human or animal subjects for the purpose of treating diseases or disorders, and for cosmetic, reconstructive or research and development purposes.

US7320325B2 discloses an apparatus and treatment method for creating intrauterine adhesions resulting in amenorrhea. In particular, the apparatus relates to an easily deployed intrauterine implant that reduces or eliminates abnormal intrauterine bleeding. In addition, the apparatus is also used as a uterine marker device for visualizing endometrial tissue thickness and potential changes. The method of the invention serves as a supplement to or a replacement for conventional hysterectomy or ablation/resection procedures used to treat menorrhagia.

US20040122463A1 discloses a device and method for dilating a cervical canal. The device includes an elongated member with an expandable anchoring component attached to its distal tip. An expandable member is attached to the elongated member proximally of the anchoring component for dilating the cervical canal. The anchoring component can be expanded after insertion of the device into the cervical canal to correctly position the device relative to the canal. The document discloses also a device and method for sealing a cervical canal. The device includes a tube having an expandable seal assembly attached to its distal end. The seal assembly has a ridged or corrugated surface when it is in an expanded condition to provide a seal for the cervical canal.

### Summary of the invention

Neither of the above solutions ensure proper positioning of the vagina or uterus during magnetic resonance imaging or thermal ablation procedures, therefore it is an object of the present invention to provide a manipulator that will enable this. A manipulator according to the present invention comprises an insert, a base and a displacement mechanism. The displacement mechanism comprises an at least one vertical member, a main member, an at least two positioning pins, a vertical pusher, an angular pusher, a first arm, a second arm, a third arm, a fourth arm, and a handle. Each vertical member includes an at least two longitudinal grooves, essentially perpendicular to the base, and a first end of the vertical pusher is attached to the each vertical member. The main member is pivotally connected to the first arm, at a first pivot point, and to the second arm at a second pivot point, and also the main member is connected to a second end of the vertical pusher and to a first end of the angular pusher. The first arm is further pivotally connected to a second end of the angular pusher, wherein the second end of the angular pusher is attached to the first arm at a point other than the first pivot point, to the third arm, at a third pivot point, and to the fourth arm, at a sixth pivot point. The second arm is further pivotally connected to the third arm, at a fourth pivot point. The third arm is further pivotally connected to the handle at a fifth pivot point. The fourth arm is further pivotally connected to the handle at a seventh pivot point, wherein the distances between the first pivot point and the third pivot point, the third pivot point and the sixth pivot point, the second pivot point and the fourth pivot point, the fifth pivot point and the seventh pivot point are essentially the same. The distances between the first pivot point and the second pivot point, and the third pivot point and the fourth pivot point are the same, and the distances between the third pivot point and the fifth pivot point, as well as between the sixth pivot point and the seventh pivot point are the same. The main member is connected to the at least two positioning pins, with each of the positioning pins being located in a different groove. The handle is connected to the insert. The centre of rotation of the insert is located proximally of the connection of the insert to the handle. The centre of rotation of the insert is positioned relative to the second pivot point such as the fifth pivot point is positioned relative to the fourth pivot point. The manipulator is made entirely of paramagnetic material. Preferably, the second end of the angular pusher is attached to the third arm at the third pivot point and the sixth pivot point.

Preferably, the main member is pivotally connected to the second end of the vertical pusher.

Preferably, the main member includes a main portion of the main member and at least one additional portion of the main member.

Preferably, the main member includes two additional portions, where all additional portions are connected to the main member.

Preferably, the second end of the vertical pusher and the first end of the angular pusher are connected to the at least one additional portion of the main member. Preferably, the second end of the vertical pusher and the first end of the angular pusher are located between the additional portions of the main member. Preferably, the manipulator includes two vertical members.

Preferably, the base includes a slide rail to which the displacement mechanism is attached, wherein the slide rail has a movement locking mechanism.

Preferably, the movement locking mechanism is a screw.

Preferably, at least one of the vertical pusher or the angular pusher is a screw. Preferably, the insert is releasably connected to the handle.

Preferably, the handle has a retaining seat that engages with the insert. Preferably, the manipulator is made of polyacetal, polytetrafluoroethylene or POM-C.

Preferably the base has a length and a width of less than 700 mm.

Preferably, the deflection angle of the insert in the sagittal plane is between -70° and +70° from the frontal plane of the patient.

Preferably, the height of the insert's centre of rotation relative to the base is between 30 and 150 mm.

Preferably, the base has a longitudinal recess at the side edges.

### Description of an advantageous embodiment of the invention

The embodiment of the invention is presented in the attached Drawings, wherein:
Fig. 1 shows a general view of the manipulator.
Fig. 2 shows a detailed view of the displacement mechanism.
Fig. 3 shows a detailed view of the displacement mechanism with visible main portion of the main member, the first arm and the second arm.

Thermal ablation is a procedure consisting in coagulation of tissues with the use of two focused ultrasound beams, which additionally interfere with each other so as to locally generate a temperature above 70°C, in which proteins are denatured and tissues are destroyed. During treatments with the use of a focused ultrasound beam (HiFu) for the thermal ablation process, which are performed with the use of magnetic resonance for real-time imaging of the course of the procedure, it is often necessary to move the uterus closer to the ultrasound beam emitter and immobilize the organs on which the treatment is performed. Such a procedure is performed without surgical intervention and disturbance of the continuity of the external tissues of patients, and consequently it is very difficult to change the position of internal organs and immobilize them permanently.

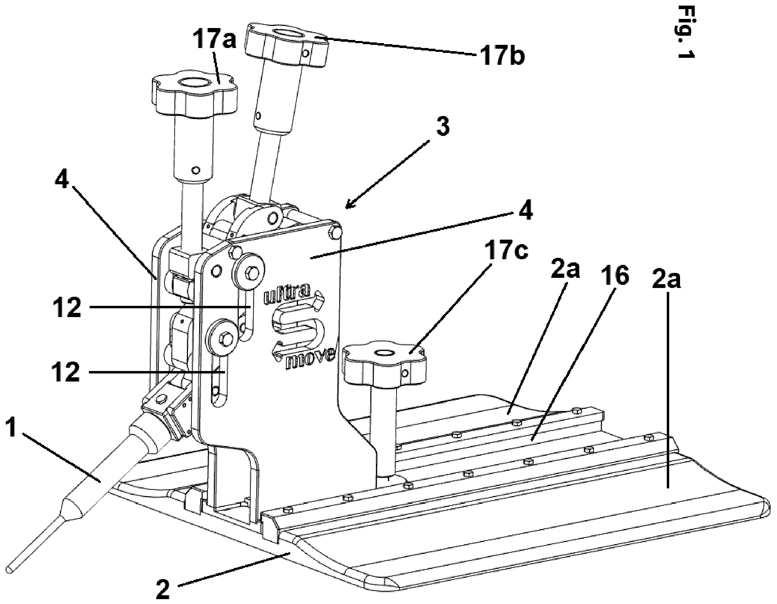

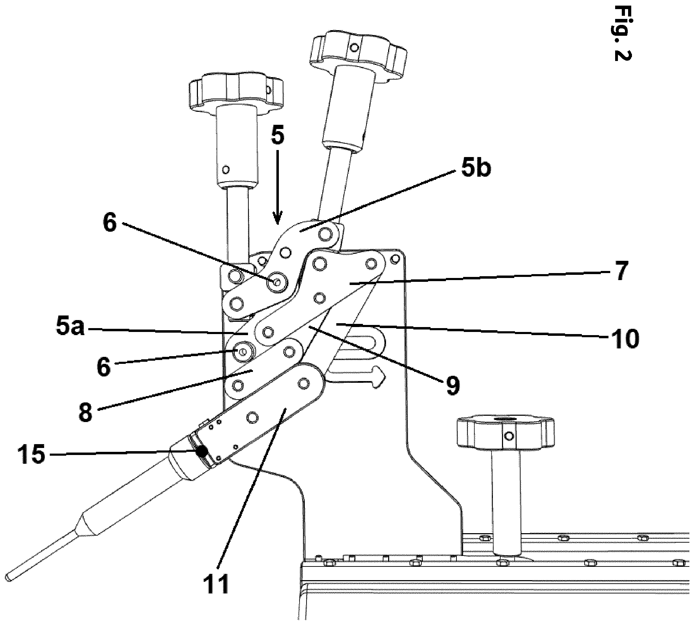

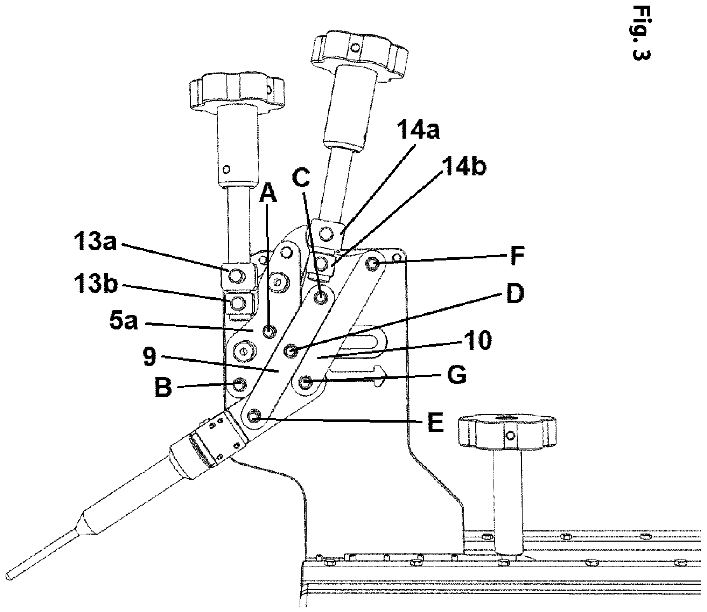

The manipulator according to the invention will now be described in detail with reference to the reference symbols in Figs. 1-3.

The manipulator comprises an insert 1, a base 2 and a displacement mechanism 3. The displacement mechanism 3 enables the vertical position of the insert 1 and the angle between the axis of the insert and the patient's sagittal plane to be adjusted. The displacement mechanism 3 comprises at least one vertical member 4, a main member 5, at least two positioning pins 6, a vertical pusher, an angular pusher, a first arm 7, a second arm 8, a third arm 9, a fourth arm 10 and a handle 11.

The vertical member 4 includes at least two longitudinal grooves 12, essentially perpendicular to the base 2, which interoperate with the positioning pins 6. This solution allows the displacement mechanism 3 to be moved only vertically. A first end 13a of the vertical pusher is attached to each vertical member 4, thereby allowing the displacement mechanism 3 to be moved vertically.

The main member 5 is pivotally connected to the first arm 7, at a first pivot point A, and to the second arm 8, at a second pivot point B, and also the main member 5 is connected to the second end 13b of the vertical pusher, and to the first end 14a of the angular pusher.

The first arm 7 is further pivotally connected to the second end 14b of the angular pusher, wherein the second end 14b of the angular pusher is attached to the first arm 7 at a point other than the first pivot point A. This attachment allows the insert 1 to be angularly adjusted. The farther the second end 14b of the angular pusher is attached from the first pivot point A, the easier and more accurately the inclination angle of the insert 1 can be changed. Preferably, the second end 14b of the angular pusher is attached to the third arm 9 at a third pivot point C and a sixth pivot point F.

The first arm 7 is also connected to the third arm 9, at the third pivot point C, and to the fourth arm 10, at the sixth pivot point F.

The second arm 8 is further pivotally connected to the third arm 9, at a fourth pivot point D.

The third arm 9 is further pivotally connected to the handle 11, at a fifth pivot point E.

The fourth arm 10 is further pivotally connected to the handle 11, at the seventh pivot point G.

The distances between the first pivot point A and the third pivot point C, the third pivot point C and the sixth pivot point F, the second pivot point B and the fourth pivot point D, the fifth pivot point E and the seventh pivot point G are essentially the same.

The distances between the first pivot point A and the second pivot point B, and between the third pivot point C and the fourth pivot point D are the same, and the distances between the third pivot point C and the fifth pivot E, as well as between the sixth pivot point F and the seventh pivot point G are the same.

The main member 5 is connected to at least two positioning pins 6, with each of the positioning pins 6 being located in a different groove 12.

The handle 11 is connected to the insert 1. The centre of rotation 15 of the insert 1 is located proximally of the connection of the insert 1 to the handle 11. The centre of rotation 15 of the insert 1 is positioned relative to the second pivot point B such as the fifth pivot point E is positioned relative to the fourth pivot point D. The above-described mechanism, comprising the main member 5, the first arm 7, the second arm 8, the third arm 9, the fourth arm 10 and the handle 11, allows to maintain a constant point of rotation 15 of the insert 1. This is important for anatomical reasons - the fixed point of rotation allows for safe displacement of genital organs.

The manipulator is made entirely of material paramagnetic under magnetic resonance imaging conditions. Due to a strong magnetic field, it is necessary to use materials that are not affected by the magnetic field or the magnetic field effect is negligible, whereby it is possible to use the manipulator without risk to the patient. In one embodiment, the main member 5 is pivotally connected to the second end 13b of the vertical pusher.

In another embodiment, the main member 5 comprises a main portion 5a of the main member 5 and at least one additional portion 5b of the main member 5. This solution allows for easier assembly and fabrication of the main member 5. Preferably, the main member 5 comprises two additional portions 5b, wherein all additional portions 5b are connected to the main member 5a. Two additional portions 5b strengthen the structure of the displacement mechanism 3.

The second end 13b of the vertical pusher and the first end 14a of the angular pusher are connected to at least one additional portion 5b of the main member 5. This solution facilitates the assembly of the manipulator and simplifies the parts, which in turn reduces both production cost and time. Preferably, the second end 13b of the vertical pusher and the first end 14a of the angular pusher are located between the additional portions 5b of the main member 5, which additionally increases the rigidity and strength of the manipulator.

In yet another embodiment, the manipulator comprises two vertical members 4, which results in a more stable manipulator structure.

In yet another embodiment, the base 2 includes a slide rail 16 to which the displacement mechanism 3 is attached, wherein the slide rail 16 has a movement locking mechanism. This solution simplifies positioning of the manipulator before the procedure.

The movement locking mechanism is a screw 17c. It should be noted that one skilled in the art will be able to propose other movement blocking mechanisms as well.

In another embodiment, at least one of the vertical pusher or angular pusher is a screw 17a, 17b. It should be noted that one skilled in the art will be able to propose also other solutions enabling the adjustment of the displacement mechanism 3, both vertically and angularly.

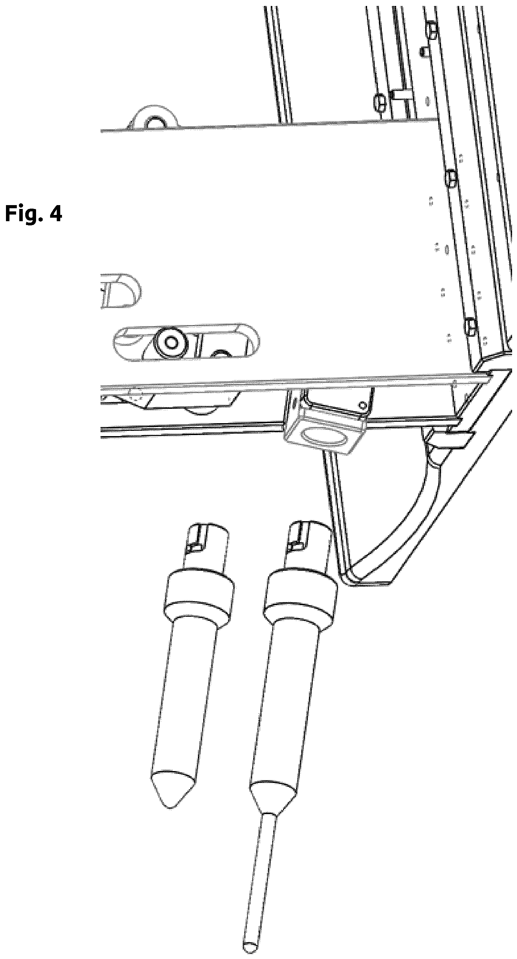

The insert 1 is releasably connected to the handle 11. Due to the anatomical differences and the area in which the treatments are performed, the insert 1 is selected according to the patient's needs. In addition, the replaceable insert 1 allows to keep the equipment used in a safe and sterile condition thanks to the possibility of replacing the insert 1 after each use without the need to wash the entire device. Preferably, the handle 11 has a retaining seat that engages with the insert 1 as shown in Fig. 4.

In a further embodiment of the invention, the manipulator is made of polyacetal, polytetrafluoroethylene or POM-C. These materials are approved for use in the conditions prevailing in the area of magnetic resonance imaging and do not interfere with the examination.

In yet another embodiment, the base has a length and a width of less than 700 mm. These dimensions allow the manipulator to be inserted into the area of magnetic resonance examination.

In a further embodiment, the deflection angle of the insert in the sagittal plane is between -70° and + 70° from the frontal plane of the patient. This range of adjustment is sufficient to properly position the organs for the purpose of the thermal ablation treatment.

In another embodiment, the height of the centre of rotation 15 of the insert 1 with respect to the base 2, considered as the base surface 2, on which the manipulator rests under normal operating conditions, wherein it is the lower part of the base in Figs. 1-3, is 30 to 150 mm. This range of adjustment is sufficient to perform a thermal ablation treatment.

In yet another embodiment, the base 2 has a longitudinal recess 2a at its side edges. The recesses 2a allow for placing patient's legs on the base 2 in a comfortable position, so that the manipulator is immobilized in respect to the patient, which improves the safety of the device operation and minimizes the chance of a device displacement during the procedure.

### List of reference symbols

- 1 -: insert
- 2 -: base
- 2a -: longitudinal recess of the base
- 3 -: displacement mechanism
- 4 -: vertical member
- 5 -: main member
- 5a -: main portion of the main member
- 5b -: additional portion of the main member
- 6 -: positioning pin
- 7 -: first arm
- 8 -: second arm
- 9 -: third arm
- 10 -: fourth arm
- 11 -: handle
- 12 -: groove
- 13a -: first end of the vertical pusher
- 13b -: second end of the vertical pusher
- 14a -: first end of the angular pusher
- 14b -: second end of the angular pusher
- 15 -: centre of rotation of the insert
- 16 -: slide rail
- 17a, 17b, 17c -: screw
- A -: first pivot point
- B -: second first pivot point
- C -: third first pivot point
- D -: fourth first pivot point
- E -: fifth first pivot point
- F -: sixth first pivot point
- G -: seventh first pivot point

## Claims

1. A manipulator including an insert (1), **characterized in that** it comprises a base (2) and a displacement mechanism (3), wherein the displacement mechanism (3) comprises an at least one vertical member (4), a main member (5), an at least two positioning pins (6), a vertical pusher, an angular pusher, a first arm (7), a second arm (8), a third arm (9), a fourth arm (10), and a handle (11), wherein the each vertical member (4) includes an at least two longitudinal grooves (12), essentially perpendicular to the base (2), and a first end (13a) of the vertical pusher is attached to the each vertical member (4), wherein the main member (5) is pivotally connected to the first arm (7), at a first pivot point (A), and to the second arm (8), at a second pivot point (B), and also the main member (5) is connected to a second end (13b) of the vertical pusher, and to a first end (14a) of the angular pusher, the first arm (7) is further pivotally connected to a second end (14b) of the angular pusher, wherein the second end (14b) of the angular pusher is attached to the first arm (7) at a point other than the first pivot point (A), to the third arm (9), at a third pivot point (C), and to the fourth arm (10), at a sixth pivot point (F),
the second arm (8) is further pivotally connected to the third arm (9), at a fourth pivot point (D),
the third arm (9) is further pivotally connected to the handle (11), at a fifth pivot point (E),
the fourth arm (10) is further pivotally connected to the handle (11) at a seventh pivot point (G), wherein
the distances between the first pivot point (A) and the third pivot point (C), the third pivot point (C) and the sixth pivot point (F), the second pivot point (B) and the fourth pivot point (D), the fifth pivot point (E) and the seventh pivot point (G) are essentially the same, wherein
the distances between the first pivot point (A) and the second pivot point (B), and between the third pivot point (C) and the fourth pivot point (D) are the same, and the distances between the third pivot point (C) and the fifth pivot point (E), as well as between the sixth pivot point (F) and the seventh pivot point (G) are the same,
wherein the main member (5) is connected to the at least two positioning pins (6), with each of the positioning pins (6) being located in a different groove (12),
whereas the handle (11) is connected to the insert (1),
wherein the centre of rotation (15) of the insert (1) is located proximally of the connection of the insert (1) to the handle (11), wherein the centre of rotation (15) of the insert (1) is positioned relative to the second pivot point (B) such as the fifth pivot point (E) is positioned relative to the fourth pivot point (D), wherein the manipulator is made entirely of a paramagnetic material.

2. The manipulator according to claim 1, **characterized in that** the second end (14b) of the angular pusher is attached to the third arm (9) at the third pivot point (C) and the sixth pivot point (F).

3. The manipulator according to either claim 1 or 2, **characterized in that** the main member (5) is pivotally connected to the second end (13b) of the vertical pusher.

4. The manipulator according to any claim 1-3, **characterized in that** the main member (5) includes a main portion (5a) of the main member (5) and at least one additional portion (5b) of the main member (5).

5. The manipulator according to claim 4, **characterized in that** the main member (5) includes two additional portions (5b), wherein all additional portions (5b) are connected to the main member (5a).

6. The manipulator according to claim 5, **characterized in that** the second end (13b) of the vertical pusher and the first end (14a) of the angular pusher are connected to at least one additional portion (5b) of the main member (5).

7. The manipulator according to claim 6, **characterized in that** the second end (13b) of the vertical pusher and the first end (14a) of the angular pusher are located between the additional portions (5b) of the main member (5).

8. The manipulator according to any claim 1-7, **characterized in that** it includes two vertical members (4).

9. The manipulator according to any claim 1-8, **characterized in that** the base (2) includes a slide rail (16) to which the displacement mechanism (3) is attached, wherein the slide rail (16) has a movement locking mechanism, preferably in a form of a screw (17c).

10. The manipulator according to any claim 1-09 **characterized in that** at least one of the vertical pusher or the angular pusher is a screw (17a, 17b).

11. The manipulator according to any claim 1-10, **characterized in that** the insert (1) is releasably connected to the handle (11).

12. The manipulator according to any claim 1-11, **characterized in that** the handle (11) has a retaining seat that engages with the insert (1).

13. The manipulator according to any claim 1-12, **characterized in that** the base has a length and a width of less than 700 mm.

14. The manipulator according to any claim 1-13, **characterized in that** the deflection angle of the insert in the sagittal plane is between -70° and +70° from the frontal plane of the patient.

15. The manipulator according to any claim 1-14, **characterized in that** the height of the centre of rotation (15) of the insert (15) relative to the base is between 30 and 150 mm.
